# FASCICULE DE BREVET EUROPEEN

(11) **EP 0 480 109 B1**
(45) Date de publication et mention de la délivrance du brevet: **04.01.1995**
(21) Numéro de dépôt: 90402802.4
(22) Date de dépôt: 09.10.1990
(51) Int. Cl.: A61D 19/02

(54) **Tubes ou paillettes pour la conservation cryogénique d'échantillons biologiques tels que virus, et procédé de remplissage**
Schlauch oder Paillette für die kryogenische Aufbewahrung eines biologischen Musters wie Virus, und Verfahren für die Einfüllung
Tube or spangle for the cryogenic conservation of biological samples like virus, and procedure for filling

(43) Date de publication de la demande: 15.04.1992
(73) Titulaire: Cassou, Robert, F-18700 Aubigny-sur-Nère (FR); Cassou, Maurice, F-61300 L'Aigle (FR); Cassou, Bertrand, F-61300 L'Aigle (FR)
(72) Inventeur: Cassou, Robert, F-18700 Aubigny-sur-Nère (FR); Cassou, Maurice, F-61300 L'Aigle (FR); Cassou, Bertrand, F-61300 L'Aigle (FR)
(74) Mandataire: CABINET BONNET-THIRION

(56) Documents cités:
- EP-A- 0 304 358

## Description

L'invention se rapporte à un tube, dit paillette, pour la conservation cryogénique d'échantillons biologiques, notamment cultures virales, formé d'un tronçon d'enveloppe tubulaire en matériau polymère sensiblement transparent, biologiquement neutre, muni d'un scellement à chacune de ses deux extrémités et comportant, au voisinage d'une première extrémité, un obturateur glissant comprenant un gel aqueux entre deux tampons en matériau élastique poreux.

L'invention se rapporte également à un procédé de remplissage d'un tube ou paillette de ce genre.

Ce type de paillettes constitué d'un tronçon d'enveloppe d'une douzaine de centimètres de long et de trois millimètres environ de diamètre, avec une épaisseur de paroi de quelques dizièmes de millimètres, est connu de longue date: l'obturateur glissant, dit aussi bouchon tripartite, a été décrit dans le document FR-A-995 878 du 20 Septembre 1946. Depuis, divers perfectionnements lui ont été apportés, qui ont porté essentiellement sur les scellements d'extrémité, en conjonction avec le matériau polymère dont est extrudée l'enveloppe. En effet, le processus d'obturation doit donner une étanchéité efficace, ne pas présenter de risques excessifs de détérioration pour l'échantillon biologique contenu et être adapté, au moins pour certaines applications, aux manipulations nécessaires à ces applications (conservation et utilisation de gamètes, fécondation in vitro).

Par ailleurs, le choix de matériau d'enveloppe a été guidé par des considérations de compatibilité avec les substances biologiques, de transparence et de facilité de mise en forme. Pratiquement seront exclus tous les matériaux qui comportent des plastifiants susceptibles de migrer.

Le document FR-A-2 128 374 a décrit un scellement par soudage à ultrason de paillettes en matériau non précisé. Le soudage est exécuté entre les mâchoires en Vés complémentaires, l'une reliée au générateur d'ultrasons et l'autre formant enclume ; on obtien ainsi un scellement qui s'inscrit dans le cylindre qui épouse le tube à l'extérieur.

Le document FR-A-2 592 797 décrit un processus d'obturation par injection d'une matière thermofusible à l'état pâteux à l'intérieur de l'extrémité de tube ; la paillette est en polychlorure de vinyle et la matière thermofusible est un mélange d'une polyoléfine et d'un copolymère d'éthylène et d'acétate de vinyle.

Le document FR-A-2 618 452 décrit une paillette obturée, à l'extrémité opposée à celle qui avoisine l'obturateur glissant, par un bouchon en élastomère capable d'être transpercé par une aiguille, et de rester étanche après que l'aiguille ait été retirée. Le bouchon en élastomère est soit introduit à force, soit formé in situ par vulcanisation d'une composition appropriée.

Le document FR-A-2 623 205 décrit une paillette pour fécondation qui ne comporte pas d'obturateur glissant, et est munie à chaque extrémité d'un bouchon en élastomère autoscellant.

Les progrès de la microbiologie ont entraîné une extension de l'usage de telles paillettes, en corrélation avec des travaux portant sur des échantillons biologiques parfois rares, et souvent notamment dans le cas de cultures virales, dangereux. Il devenait impératif de réviser la conception de la paillette, et notamment de ses scellements d'extrémités, pour d'une part éviter l'utilisation d'aiguilles d'injection, susceptibles de provoquer des blessures pour le personnel appelé à les manipuler, et faciliter l'automatisation des opérations de remplissage et de reprise des échantillons, et d'autre part limiter sévèrement les risques de perte d'étanchéité des paillettes remplies, sous l'effet des contraintes et chocs thermiques liés à la congélation par immersion dans l'azote liquide, et la décongélation rapide.

Bien que les paillettes actuelles présentent un taux de défaut, dans les conditions cryogéniques, qui soit acceptable pour des travaux courants, ce taux de défaut devient excessif dans la mesure où la mise hors service d'une paillette remplie peut faire échouer un programme de recherche ou que les risques d'infection dus à la fuite d'un échantillon présentent un caractère dramatique.

Les demandeurs se sont donc fixés pour but de réaliser un tube ou paillette qui présenterait une fiabilité considérablement accrue en conservation cryogénique, y compris dans les zones de scellement et qui pourrait être rempli et scellé aisément sur un montage automatique qui ne ferait pas intervenir une manipulation d'un opérateur.

Et les demandeurs estiment avoir atteint ce but en proposant un tube dit paillette pour la conservation cryogénique d'échantillons biologiques, notamment culture virale, formé d'un tronçon d'enveloppe tubulaire calibrée en matériau polymère sensiblement transparent, biologiquement neutre, muni d'un scellement à chacune de ses deux extrémités et comportant au voisinage d'une première extrémité, un obturateur glissant comprenant un gel aqueux entre deux tampons en matériau élastique poreux, caractérisé en ce que le matériau polymère est une résine ionomère, les scellements d'extrémité étant formés par soudage autogène du tube sur une étendue axiale limitée.

Les résines ionomères, formées par association d'un copolymère d'éthylène et d'un acide carboxylique avec un cation métallique possédent la propriété de se comporter, au dessus d'une zone de température de transition, située dans la gamme 40-90°, comme un matériau thermoplastique, tandis qu'en dessous de cette zone de transition elles se comportent comme un matériau réticulé, les cations métalliques formant des liaisons transversales entre des chaînes linéaires de copolymère. La transformation est réversible. Le soudage autogène du tube est simple et efficace, au-dessus des températures de transition ; le refroidissement après soudage n'induit que peu de tensions internes, le figeage de la résine par réticulation ionique ne s'accompagnant pas de variations importantes de volume.

De telles résines ionomères ont été décrites dans les brevets français 1 336 164, 1 350 608 et 1 430 478, déposés par la Sté Dupont de Nemours respectivement les 28 Août 1962, 23 Janvier 1963 et 26 Février 1965. Ces résines sont commercialisées sous le nom de marque "Surlyn".

La plupart des solides possèdent une température de fragilisation en dessous de laquelle le fluage est pratiquement inhibé. Les matières polymères thermoplastiques sont, en général, très sensibles à la fragilisation au froid, notamment quand elles ne comportent pas de plastifiant. Or les résines ionomères possédent des températures de fragilisation très basses, toujours inférieures à 213K, et pour la plupart inférieure à 173 K. Pour certaines résines "Surlyn" on n'a pas pu mettre en évidence de température de fragilisation.

On comprend que les manipulations des paillettes classiques, refroidies à la température de l'azote liquide, 77 K, sont délicates, et se prêtent mal à des manipulations automatiques. En outre si des tensions internes subsistent dans les soudures, il peut se produire des fêlures dans le scellement. Ces difficultés sont atténuées ou disparaîssent avec les paillettes selon l'invention.

La structure réticulée des résines "Surlyn" à la température ambiante confère aux paillettes une tenue mécanique intéressante ; les paillettes n'ont pas tendance à fluer sous leur propre poids et restent rectilignes. La position des extrémités des paillettes, posées sur un support convenable, sera précise, et l'introduction d'une aiguille ou d'une buse conique pour le remplissage est ainsi rendue aisée, sans que l'intervention d'opérateurs, même occasionnelle, soit nécessaire.

On remarquera que pendant longtemps, les paillettes classiques ont donné satisfaction, les pertes d'étanchéité par bris ou fissures dues à la fragilation apparaissant compatibles avec le taux acceptable des erreurs et accidents de manipulation inévitables. Ce n'est que récemment que la nécessité d'abaisser les seuils de taux de déchets acceptables à conduit la Demanderesse à analyser les causes d'accidents possibles, pour pallier ces accidents. On peut supposer qu'à l'origine, les fabricants de paillettes ont choisi les matériaux usuels dans l'industrie des matériels de laboratoires biologiques, dont le comportement, tant vis-à-vis des échantillons biologiques que des utilisations cryogéniques, était connu.

Bien entendu les résines "Surlyn" possèdent des qualités de transparence et de neutralité biologique convenables. On notera accessoirement que ces résines acceptent les encres à marquer, ce qui permet d'identifier les paillettes remplies, sans risque d'erreur.

De préférence la résine ionomère sera du type commercialisé sous la dénomination "Surlyn" 8921. Cette résine comporte un cation métallique sodium, et l'on n'a pu en déterminer une température de fragilisation. En relation avec la zone de transition, la température de fléchissement sous charge, de 0,46 MPa est de 45°C, la température VICAT (vitesse B) de 58°C, la température de fusion 84°C, et la température de solidification de 52°C.

Sous un autre aspect, l'invention propose un procédé de remplissage d'un tube dit paillette pour la conservation cryogénique d'échantillons biologiques, notamment culture virale, constitué d'un tronçon d'enveloppe tubulaire calibrée en matériau polymère sensiblement transparent, biologiquement neutre, procédé où l'on met en place un obturateur glissant à proximité d'une première extrémité du tube en insérant successivement un premier tampon élastique poreux, une dose de poudre apte à gélifier au contact d'une phase aqueuse, puis un second tampon élastique poreux, l'obturateur étant poussé au-delà d'une zone du tube réservée au scellement de la première extrémité, on injecte dans le tube, préalablement stérilisé, l'échantillon biologique à travers la seconde extrémité et au-delà d'une zone réservée au scellement de cette extrémité, tandis que l'atmosphère du tube est évacuée à travers l'obturateur et la première extrémité jusqu'à ce que la phase aqueuse, traversant le premier tampon provoque la gélification de la poudre et partant l'étanchéité de l'obturateur, caractérisé en ce que, l'enveloppe tubulaire étant constituée d'une résine ionomère, on scelle les extrémités du tube en écrasant le tube dans les zones réservées en sorte d'appliquer l'une contre l'autre deux parties de la couche périphérique interne du tube, et en portant au moins ces deux parties à une température suffisante pour qu'elles fusionnent.

L'utilisation d'une résine ionomère pour constituer l'enveloppe tubulaire, outre les avantages spécifiques apportés par la résine au tube dit paillette pour la conservation cryogénique d'échantillon biologique, permet d'effectuer le scellement de façon simple et facile à automatiser, sans risquer de détériorer le contenu par surchauffe de l'échantillon, tout en assurant la stérilisation du joint de soudure proprement dit, en raison de la température à laquelle le soudage autogène est effectué (dans la gamme 90°C-110°C).

Des caractéristiques secondes et les avantages de l'invention ressortiront d'ailleurs de la description qui va suivre, à titre d'exemple, en référence aux dessins annexés dans lesquels :
la figure 1 est une vue, partiellement coupée, d'un tube ou paillette selon l'invention ;
la figure 2A est une vue d'un premier processus de remplissage d'une paillette ;
la figure 2B est une vue d'une variante de remplissage;
la figure 3A illustre un processus de scellement d'une paillette ;
la figure 3B est une coupe longitudinale d'une paillette scellée et remplie ;
la figure 4 représente schématiquement en élévation un banc de remplissage et scellement de paillettes ;
la figure 5 est une vue de dessus du banc de la figure 4 ;
les figures 6A et 6B sont des vues dans deux plans orthogonaux, d'un premier type de scellement ;
les figures 7A et 7B sont des représentations analogues d'un deuxième type de scellemeent ;
les figures 8A et 8B sont des représentations analogues d'un troisième type de scellement.

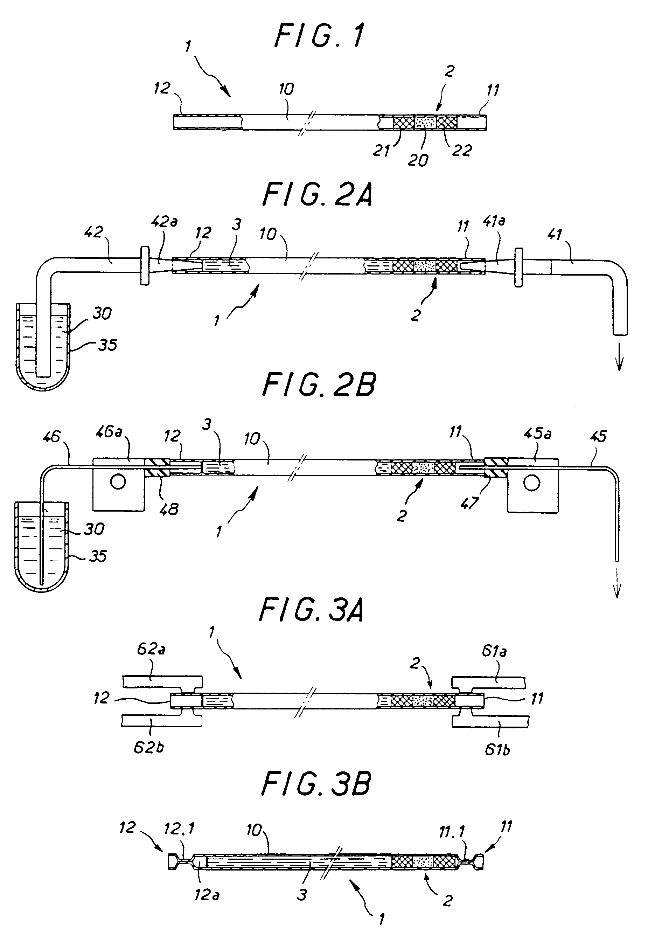

Selon la forme de réalisation choisie et représentée à la figure 1, un tube 1, dit paillette, est prévu pour la conservation cryogénique, à la température de l'azote liquide, soit 77 K, d'échantillons biologiques, notamment cultures virales. Il est conçu pour assurer une haute sécurité contre des fuites d'échantillons, malgré les contraintes apportées par les basses températures, et pour permettre des manipulations automatiques notamment au remplissage, afin de minimiser les risques d'infection au personnel utilisateur. Ce tube 1 comporte une enveloppe tubulaire 10, exécutée par extrusions d'une résine ionomère commercialisée par la Société Dupont de Nemours sous le nom de marque "Surlyn" 8921. On a évoqué plus haut les propriétés de cette résine, et notamment son absence de point de fragilisation à 77 k et au-dessus.

Le tube présente une longueur, avant remplissage, de 90 mm un diamètre extérieur de 3 mm environ et un diamètre intérieur de 2,5 mm.

Classiquement le tube 1 contient un obturateur glissant 2, à proximité d'une extrémité 11, qui sera dite première extrémité. Cet obturateur comprend, de part et d'autre d'une dose 20 de poudre d'alcool polyvinylique, hydrophile et capable de se gélifier au contact d'une phase aqueuse, deux tampons poreux 21 et 22, constitués de tronçons de mêche de coton tressé. La longueur de l'obturateur est d'environ 18 mm, chacun des éléments 20, 21 et 22 occupant une longueur d'environ 6 mm. Le volume intérieur utile est de 250 µl. Le calibrage de l'enveloppe tubulaire 10 à l'extrusion , et notamment le calibrage de la section intérieure permet la reproductibilité du diamètre intérieur.

Le remplissage de la paillette est illustré à la figure 2A. Le tube 1, qui a été préalablement stérilisé, est pris entre deux canules tronconiques 41a et 42a; qui pénètrent respectivement dans les extrémités 11 et 12; la forme tronconique des canules 41a et 42a, jointe à un effort de serrage l'une vers l'autre, assure l'étanchéité du raccordement. La canule 41a, à l'extrémité d'une canalisation 41, est reliée à un réservoir en dépression, tandis que la canule 42a est à l'extrémité d'une canalisation 42 qui plonge dans un tube à essai 35, contenant l'échantillon biologique 30.

L'atmosphère du tube 1 est évacuée à travers l'obturateur glissant 2, et l'échantillon biologique 30 est aspiré à travers le tube 42 et pénètre dans l'enveloppe tubulaire 10 jusqu'à atteindre l'obturateur 2. L'échantillon biologique 3 s'infiltre dans le tampon 21 et vient hydrater la poudre 20, qui forme un gel imperméable par hydratation. Le remplissage est ainsi arrêté. Lorsque les canules 41a et 42a sont reculées, l'échantillon biologique 3 reste sous forme d'une colonne continue et l'extrémité 12, sur la profondeur de pénétration de la canule 42a ne contient pas d'échantillon et reste disponible pour le scellement ultérieur, comme l'extrémité 11, qui n'a pas été atteinte par l'échantillon.

On appréciera que la rigidité relative du tube 1 en "Surlyn" à température ambiante autorise une mise en compression longitudinale de l'enveloppe 10, nécessaire à l'étanchéité, et que le maintien d'une forme précise sans flexion, en dessous de la zone de transition de la résine ionomère, permet l'introduction automatique précise des canules 41a et 42a dans les extrémités 11 et 12 de la paillette 1.

Le dispositif représenté à la figure 2B est analogue à celui de la figure 2A, à l'exception toutefois que les tubes 41 et 42 sont remplacés par des tubes capillaires 45 et 46, fixés dans des pinces 45a et 46a. L'étanchéité entre tubes 45 et 46 et extrémités 11 et 12 est assurée par des bouchons en élastomère 47 et 48, enfilés sur les tubes capillaires 45 et 46, et portant en bout sur les tranches d'extrémités 11 et 12 de la paillette 1. Le processus de remplissage n'est pas modifié. On notera toutefois que le tube capillaire 46 pénètre suffisamment profondément dans l'extrémité 12 de la paillette 1 pour que cette extrémité 12 reste vide sur une longueur correspondant sensiblement à la profondeur d'enfoncement du tampon 22 dans l'extrémité 11.

La paillette 1, remplie d'échantillon biologique 3 est alors scellée comme il est illustré à la figure 3A.

Les extrémités 11 et 12 sont serrées entre des paires de mâchoires 61a, 61b; 62a, 62b, jusqu'à écrasement de l'enveloppe tubulaire suffisant pour que les parties opposées des surfaces intérieures des extrémités 11 et 12 viennent en contact. Les paires de mâchoires 61a, 61b ; 62a, 62b sont chauffées à une température telle que la résine ionomère fonde entre les mâchoires et que les parties opposées en contact fusionnent.

On rappelle que la résine ionomère choisie présente une température de fusion de 84°C, et que la zone de transition entre l'état thermofusible et l'état réticulé se situe entre 45° et 60°C, à peu près.

Après scellement, la paillette 1 présente sensiblement l'aspect indiqué figure 3B, avec aux deux extrémités 11 et 12 des zones aplaties de scellement 11.1 et 12.1. On notera que entre la zone de scellement 12.1 et l'extrémité de la colonne 3 d'échantillon biologique subsiste une bulle de gaz 12a. Outre le fait que, de façon connue, la bulle de gaz 12a évite une rupture de la paillette, la congélation de l'échantillon s'accompagnant d'une augmentation de volume, lors du scellement, la bulle 12a évite un contact entre la zone chauffée 12.1 et l'échantillon biologique, qui pourrait si non être altéré.

On notera que, en revanche, si des traces d'échantillon biologique s'étaient étendues entre les extrémités 11 et 12 de la paillette, les matières organiques de ces traces seraient essentiellement détruites à la température de fusion de la résine, avec stérilisation des extrémités accessibles.

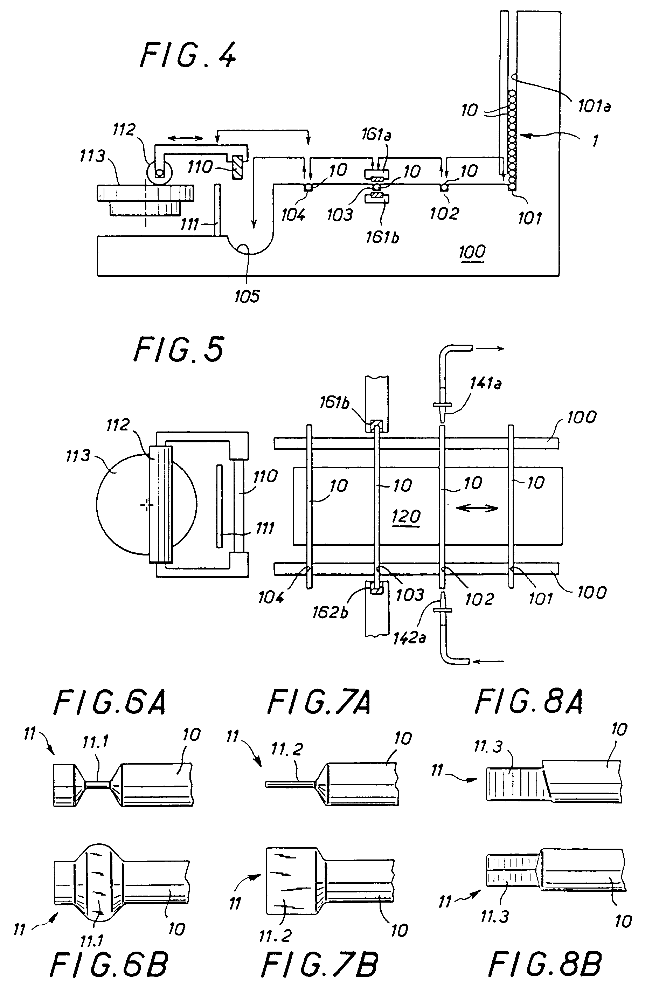

En se reportant aux figures 4 et 5, qui représentent un banc de remplissage, on voit que le banc 100 comporte des encoches 101, 102, 103 et 104, pour mettre en position les paillettes. Un organe transporteur 120 vient périodiquement soulever les paillettes, puis les déplacer vers la gauche d'une longueur entre encoches, les descendre dans l'encoche correspondante, et s'éclipser pour recommencer, après une période choisie, le transport.

Les paillettes stérilisées sont déposées dans le chargeur vertical 101 a, la paillette inférieure s'engageant dans l'encoche 101 ou encoche de chargement. Après transfert dans l'encoche 102 à la position de remplissage, des canules 141a et 142a sont rapprochées pour s'introduire dans les extrémités de la paillette, le remplissage s'effectuant comme il a été décrit en référence à la figure 2A.

Après remplissage, la paillette 10 est transférée dans l'encoche 103, où elle est soudée par des mâchoires chauffées 161a, 161b ; 162a, 162b, par le processus décrit en référence à la figure 3A.

Après cela, la paillette 10 est transférée dans l'encoche 104, où elle subit un marquage. Un marqueur 111, portant des caractères en relief, est encré par passage d'un rouleau encreur, couplé à un plateau encrier 113 tournant. Un tampon de transfert 110 reçoit, dans un mouvement de recul la marque formée par le marqueur 111, et, dans un mouvement d'avance, reporte la marque sur la paillette 10 dans l'encoche 104.

A l'action suivante de l'organe transporteur 120, la paillette 10 est évacuée dans la goulette 105.

On appréciera que toutes ces opérations sont effectuées sans intervention d'un opérateur humain.

On notera que le marquage pourrait paraître à priori une opération secondaire, sans risque pour un opérateur humain. Mais il est important que ce marquage intervienne juste après que la paillette soit personnalisée, pour éviter toute erreur. Par ailleurs, ce marquage aurait été beaucoup plus complexe à réaliser si le matériau dont est formé la paillette n'était pas susceptible de fixer l'encre de marquage.

On se reportera aux figures 6 à 8, chacune constituée d'une figure A, prise parallèlement à la direction des rapprochements des mâchoires, et d'une figure B.

Les figures 6A et 6B montrent la forme de scellement 11.1 obtenue par serrage entre des machoîres plates étroites, et qui laisse intacte la tranche d'enveloppe 10 de paillette. Cette forme sera la forme usuelle.

Les figures 7A et 7B montrent un scellement 11.2 en forme de palette, obtenu par serrage entre des mâchoires planes qui sont venues saisir la paillette en bout. Cette forme pourra être préférée pour les facilités de prise de la palette, par ailleurs cette forme supprime la cavité d'extrémité du scellement 11.1

Les figures 8A et 8B montrent un scellement 11.3 en forme de Vé, analogue à ce qui est décrit dans FR-A-2 128 374. Cette forme, résultant du serrage entre deux mâchoires en Ve, l'une saillante et l'autre rentrante, présente l'avantage que le scellement est entièrement contenu à l'intérieur du cylindre qui épouse la surface extérieure de la paillette, de sorte que la fixation de la paillette dans un mandrin est rendue plus aisée.

Bien entendu, il pourra être intéressant de conformer différemment les deux scellements d'extrémité de paillettes, pour des raisons d'opportunité. D'ailleurs d'autre forme de scellement sont concevables.

On comprend, bien entendu que, pour récupérer l'échantillon biologique aux fins d'utilisation, on sectionne les extrémités 11 et 12 au ras des zones non déformées qui sont en deçà des scellements , et qu'on chasse l'échantillon en poussant mécaniquement ou pneumatiquement l'obturateur glissant.

Il va de soi que l'invention n'est pas limitée aux exemples décrits, mais on embrasse toutes les variantes d'exécution, dans le cadre des revendications.

## Revendications

1. Tube dit paillette (1) pour la conservation cryogénique d'échantillons biologiques (3), notamment culture virale, formé d'un tronçon d'enveloppe tubulaire (10) calibrée en matériau polymère sensiblement transparent, biologiquement neutre, muni d'un scellement (11.1, 12.1) à chacune de ses deux extrémités (11, 12) et comportant au voisinage d'une première extrémité (11), un obturateur glissant (2) comprenant un gel aqueux (20) entre deux tampons (21, 22) en matériau élastique poreux, caractérisé en ce que le matériau polymère est une résine ionomère, les scellements d'extrémité (11.1, 12.1) étant formés par soudage autogène du tube (1) sur une étendue axiale limitée.

2. Tube dit paillette selon la revendication 1, caractérisé en ce que ladite résine ionomère est du Surlyn® de type 8921.

3. Tube dit paillette selon la revendication 1, caractérisé en ce que le scellement (11.1, 11.2) comporte une zone avec deux faces extérieures planes parallèles, qui s'étendent perpendiculairement à l'axe sur une largeur supérieure au diamètre extérieur courant de l'enveloppe.

4. Tube dit paillette selon la revendication 1, caractérisé en ce que le scellement (11.3) présente des faces extérieures en forme de Vés, l'une saillante et l'autre rentrante, le scellement étant entièrement contenu dans un cylindre épousant la partie courante du tube.

5. Tube dit paillette selon la revendication 1, caractérisé en ce qu'il contient un volume déterminé d'échantillon biologique (3), entre l'obturateur glissant (2) et une bulle de gaz (12a) au contact du scellement (12.1) de la seconde extrémité (12) d'enveloppe (10).

6. Procédé de remplissage d'un tube dit paillette (1) pour la conservation cryogénique d'échantillons biologiques (3), notamment culture virale, constitué d'un tronçon d'enveloppe (10) tubulaire calibrée en matériau polymère sensiblement transparent biologiquement neutre, procédé où l'on met en place un obturateur glissant (2) à proximité d'une première extrémité (11) du tube (1) en insérant successivement un premier tampon élastique poreux (21), une dose de poudre (20) apte à gélifier au contact d'une phase aqueuse, puis un second tampon élastique poreux (22), l'obturateur étant poussé au-delà d'une zone du tube réservée au scellement (11.1) de la première extrémité (11), on injecte dans le tube, préalablement stérilisé, l'échantillon biologique (30) à travers la seconde extrémité (12) et au-delà d'une zone réservée au scellement (12.1) de cette extrémité (12), tandis que l'atmosphère du tube est évacuée à travers l'obturateur (2) et la première extrémité (11) jusqu'à ce que la phase aqueuse, traversant le premier tampon (21) provoque la gélification de la poudre (20) et partant l'étanchéité de l'obturateur (2), caractérisé en ce que, l'enveloppe tubulaire (20) étant constituée d'une résine ionomère, on scelle les extrémités (11, 12) du tube en écrasant le tube dans les zones réservées en sorte d'appliquer l'une contre l'autre deux parties de la couche périphérique interne du tube, et en portant au moins ces deux parties à une température suffisante pour qu'elles fusionnent.

7. Procédé suivant la revendication 6, caractérisé en ce que la résine ionomère est du Surlyn® de type 8921.

8. Procédé suivant la revendication 6, caractérisé en ce que, pour le scellement (11.1, 12.1 ; 12.2, 12.3) des extrémités (11, 12), les zones réservées sont serrées entre des mâchoires complémentaires (61a, 61b ; 62a, 62b ; 161a, 161b ; 162a, 162b), portées à une température suffisante pour que les parties de couche interne périphérique fusionnent.

9. Procédé suivant la revendication 8, caractérisé en ce que les mâchoires (61a, 61b ; 62a, 62b ; 161a, 161b ; 162a, 162b) ont des faces de serrage planes et prallèles.

10. Procédé suivant la revendication 8, caractérisé en ce que les mâchoires sont en forme de Vés, l'une saillante et l'autre rentrante.

11. Procédé suivant la revendication 6, caractérisé en ce que l'on ménage lors du remplissage du tube (1) par l'échantillon biologique (3), une bulle de gaz (12a) entre cet échantillon (3) et la zone réservée de la seconde extrémité (12).

## Claims

1. A tube referred to as a small straw-like tube portion (1) for cryogenically preserving biological samples (3), in particular viral culture, formed by a calibrated tubular casing portion (10) of substantially transparent, biologically neutral polymer material and provided with a seal (11.1, 12.1) at each of its two ends (11, 12) and comprising in the vicinity of a first end (11) a sliding stopper (2) including an aqueous gel (20) between two plugs (21, 22) of porous elastic material, characterised in that the polymer material is an ionomer resin, the end seals (11.1, 12.1) being formed by autogenous welding of the tube (1) over a limited axial extent.

2. A tube referred to as a small straw-like tube portion according to claim 1 characterised in that said ionomer resin is Surlyn® of type 8921.

3. A tube referred to as a small straw-like tube portion according to claim 1 characterised in that the seal (11.1, 11.2) comprises a zone with two flat parallel outer faces which extend perpendicularly to the axis over a width greater than the running outside diameter of the casing.

4. A tube referred to as a small straw-like tube portion according to claim 1 characterised in that the seal (11.3) has V-shaped outer faces, one being of a projecting configuration and the other being of a re-entrant configuration, the seal being completely contained in a cylinder corresponding to the running portion of the tube.

5. A tube referred to as a small straw-like tube portion according to claim 1 characterised in that it contains a given volume of biological sample (3) between the sliding stopper (2) and a bubble of gas (12a) in contact with the seal (12.1) of the second end (12) of the casing (10).

6. A process for filling a tube referred to as a small straw-like tube portion (1) for cryogenically preserving biological samples (3), in particular viral culture, formed by a calibrated tubular casing portion (10) of substantially transparent, biologically neutral polymer material, in which process a sliding stopper (2) is set in position in the vicinity of a first end (11) of the tube (1) by successively inserting a first porous elastic plug (21), a quantity of powder (20) capable of gelling upon coming into contact with an aqueous phase, and then a second porous elastic plug (22), the stopper being pushed beyond a zone of the tube which is reserved for the seal (11.1) for the first end (11), the biological sample (30) is injected into the previously sterilised tube through the second end (12) and beyond a zone reserved for the seal (12.1) for said end (12), while the atmosphere in the tube is evacuated through the stopper (2) and the first end (11) until the aqueous phase passing through the first plug (21) causes gelling of the powder (20) and therefore sealing integrity of the stopper (2), characterised in that, the tubular casing (20) being formed by an ionomer resin, the ends (11, 12) of the tube are sealed by crushing the tube in the reserved zones in such a way as to apply two parts of the integral peripheral layer of the tube against each other and raising at least said two parts to a sufficient temperature for them to fuse.

7. A process according to claim 6 characterised in that the ionomer resin is Surlyn® of type 8921.

8. A process according to claim 6 characterised in that for producing the seals (11.1, 12.1; 12.2, 12.3) for the ends (11, 12) the reserved zones are clamped between complementary jaws (61a, 61b; 62a, 62b; 161a, 161b; 162a, 162b) which are raised to a sufficient temperature for the internal peripheral layer parts to fuse.

9. A process according to claim 8 characterised in that the jaws (61a, 61b; 62a, 62b; 161a, 161b; 162a, 162b) have flat parallel clamping faces.

10. A process according to claim 8 characterised in that the jaws are V-shaped, one being of a projecting configuration and the other being of a re-entrant configuration.

11. A process according to claim 6 characterised in that when the tube (1) is filled with the biological sample (3) a bubble of gas (12a) is provided between said sample (3) and the reserved zone of the second end (12).

## Patentansprüche

1. Rohr, welches Paillette (1) genannt wird, für die kryogenische Aufbewahrung von biologischen Mustern (3), insbesondere einer Virenkultur, welches aus einem Teilstück einer rohrförmigen Hülle (10), welche aus einem im wesentlichen transparenten, biologisch neutralen, polymeren Material kalibriert ist, welches an jedem seiner zwei Enden (11, 12) mit einer Versiegelung (11.1, 12.1) versehen ist und welches in der Nachbarschaft eines ersten Endes (11) einen Gleitverschluß (2) umfaßt, welcher ein wässriges Gel (20) zwischen zwei Stopfen (21, 22) aus elastischem, porösen Material enthält, dadurch gekennzeichnet, daß das polymere Material ein ionomeres Harz ist und daß die Versiegelungen der Enden (11.1, 12.1) durch Autogenschweißen des Rohres (1) auf einer achsial beschränkten Breite gebildet sind.

2. Rohr, welches Paillette genannt wird, nach Anspruch 1, dadurch gekennzeichnet, daß das genannte ionomere Harz aus Surlyn® der Type 8921 ist.

3. Rohr, welches Paillette genannt wird, nach Anspruch 1, dadurch gekennzeichnet, daß die Versiegelung (11.1, 11.2) eine Zone mit zwei parallelen, ebenen, äußeren Flächen aufweist, welche sich normal auf die Achse auf einer Breite, welche über jener des Außendurchmessers der Hülle liegt, erstrecken.

4. Rohr, welches Paillette genannt wird, nach Anspruch 1, dadurch gekennzeichnet, daß die Versiegelung (11.3) zwei äußere Vorderseiten in Form von Prismen aufweist, von welchen eine vorspringend und die andere zurückspringend ausgebildet ist, wobei die Versiegelung insgesamt in einem Zylinder, welcher an den fortlaufenden Bereich des Rohres angepaßt ist, enthalten ist.

5. Rohr, welches Paillette genannt wird, nach Anspruch 1, dadurch gekennzeichnet, daß es ein bestimmtes Volumen des biologischen Musters (3) zwischen dem Gleitverschluß (2) und einer Gasblase (12a), welche in Kontakt mit der Versiegelung (12.1) des zweiten Endes (12) der Hülle (10) ist, enthält.

6. Verfahren zum Befüllen eines Rohres, welches Paillette genannt wird, für die kryogenische Aufbewahrung von biologischen Mustern (3), insbesondere einer Virenkultur, bestehend aus einem Teilstück der rohrförmigen Hülle (10), welche aus einem im wesentlichen transparenten, biologisch neutralen, polymeren Material kalibriert ist, bei welchem Verfahren man einen Gleitverschluß (2) in der Nähe eines ersten Endes (11) des Rohres (1) placiert, indem sukzessive ein erster elastischer poröser Stopfen (21), eine Pulvermenge (20), welche fähig ist, im Kontakt mit einer wässigen Phase zu gelieren, dann ein zweiter elastischer, poröser Stopfen (22) eingeführt wird, wobei der Verschluß jenseits eines Bereiches des Rohres, welcher für die Versiegelung (11.1) des ersten Endes (11) reserviert ist, gedrückt wird, wobei man in das zuvor sterilisierte Rohr das biologische Muster (30) über das zweite Ende (12) und jenseits eines für die Versiegelung (12.1) dieses Endes (12) reservierten Bereiches, einbringt, während die Atmosphäre des Rohres über den Verschluß (2) und das erste Ende (11) evakuiert wird, bis die wässrige Phase, indem sie den ersten Stopfen (21) passiert, die Gelbildung des Pulvers (20) und demnach die Dichtheit des Verschlußes (2) bewirkt, dadurch gekennzeichnet, daß die rohrförmige Hülle (20) aus einem ionomeren Harz gebildet ist, daß man die Enden (11, 12) des Rohrens versiegelt, indem man das Rohr in den dafür vorgesehenen Bereichen durch gegenseitiges Aufeinanderlegen der zwei Bereiche der inneren Umfangsschicht des Rohres quetscht und indem wenigstens diese zwei Bereiche auf eine ausreichende Temperatur gebracht werden, damit sie miteinander verschmelzen.

7. Verfahren nach Anspruch 6, dadurch gekennzeichnet, daß das ionomere Harz aus Surlyn® der Type 8921 ist.

8. Verfahren nach Anspruch 6, dadurch gekennzeichnet, daß für das Versiegeln (11.1, 12.1; 12.2, 12.3) der Enden (11, 12) die dafür reservierten Bereiche zwischen komplementären Klemmen (61a, 61b; 62a, 62b; 161a, 161b; 162a, 162b) geklemmt werden, welche auf eine ausreichende Temperatur zum Verschmelzen dieser Bereiche der inneren Umfangsschicht gebracht sind.

9. Verfahren nach Anspruch 8, dadurch gekennzeichnet, daß die Klemmen (61a, 61b; 62a, 62b; 161a, 161b; 162a, 162b) ebene und parallele Klemmflächen aufweisen.

10. Verfahren nach Anspruch 8, dadurch gekennzeichnet, daß die Klemmen in Form von Prismen ausgebildet sind, wobei die eine vorspringend und die andere zurückspringend ausgebildet ist.

11. Verfahren nach Anspruch 6, dadurch gekennzeichnet, daß man bei der Befüllung des Rohres (1) durch das biologische Muster (3) eine Gasblase (12a) zwischen diesem Muster (3) und dem reservierten Bereich des zweiten Endes (12) ausspart.
